(19) Europäisches Patentamt
European Patent Office
Office européen des brevets

(11) **EP 2 139 842 B1**

(12) **EUROPÄISCHE PATENTSCHRIFT**

(45) Veröffentlichungstag und Bekanntmachung des Hinweises auf die Patenterteilung:
**05.03.2014 Patentblatt 2014/10**

(51) Int Cl.:
*C07C 57/075* (2006.01)   *C07C 57/04* (2006.01)
*C07C 51/50* (2006.01)   *C07C 51/41* (2006.01)

(21) Anmeldenummer: **08735454.4**

(22) Anmeldetag: **20.03.2008**

(86) Internationale Anmeldenummer:
**PCT/EP2008/053422**

(87) Internationale Veröffentlichungsnummer:
**WO 2008/116840 (02.10.2008 Gazette 2008/40)**

(54) **TRANSPORT EINER MONOMER-ZUSAMMENSETZUNG IN EINEM VERKEHRSMITTEL ODER EINER ROHRLEITUNG**

TRANSPORTATION OF A MONOMER COMPOSITION IN A TRANSPORT MEANS OR PIPE

TRANSPORT D'UNE COMPOSITION MONOMÉRIQUE DANS UN MOYEN DE TRANSPORT OU UNE CONDUITE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **23.03.2007 EP 07104841**

(43) Veröffentlichungstag der Anmeldung:
**06.01.2010 Patentblatt 2010/01**

(73) Patentinhaber: **BASF SE**
**67056 Ludwigshafen (DE)**

(72) Erfinder:
• **FUNK, Rüdiger**
**65527 Niedernhausen (DE)**
• **HEIDE, Wilfried**
**67251 Freinsheim (DE)**

• **WEISMANTEL, Matthias**
**63637 Jossgrund (DE)**
• **HAMMON, Ulrich**
**68163 Mannheim (DE)**
• **BENNETT, Andrea, Karen**
**68161 Mannheim (DE)**

(74) Vertreter: **Reitstötter - Kinzebach**
**Patentanwälte**
**Sternwartstrasse 4**
**81679 München (DE)**

(56) Entgegenhaltungen:
**WO-A-03/095410**

• **DATABASE WPI Week 197807 Thomson Scientific, London, GB; AN 1978-12932A XP002487934 & JP 50 142511 A (TOA GOSEI CHEM IND LTD) 17. November 1975 (1975-11-17)**

**EP 2 139 842 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende, Erfindung betrifft den Transport einer Monomer-Zusammensetzung in einem Verkehrsmittel oder einer Rohrleitung.

Wasserabsorbierende Harze, auch als Superabsorber oder SAP (Superabsorbing Polymers) bezeichnet, sind in der Lage, wässrige Flüssigkeiten unter Bildung eines Hydrogels zu absorbieren und dadurch zu binden. Superabsorber finden daher insbesondere in Hygieneartikeln wie Windeln, Inkontinenzeinlagen und -hosen, Damenbinden und dergleichen zur Absorption wässriger Körperflüssigkeiten Verwendung. Weitere Anwendungen der Superabsorber betreffen Brandschutz, Kabelummantelungen, Packungsrnaterialien und medizinische Anwendungen. Einen umfassenden Überblick über SAP, ihre Anwendung und ihre Herstellung gibt F.L. Buchholz und A.T. Graham (Herausgeber) in "Modern Superabsorbent Polymer Technology", Wiley-VCH, New York, 1998.

Unter den Superabsorbern sind solche auf Basis von Acrylsäure eine besonders wichtige Stoffklasse.

Üblicherweise wird monomere Acrylsäure zum Einsatzort der Herstellung der wasserabsorbierenden Harze transportiert. Acrylsäure ist jedoch eines der reaktivsten bekannten Vinylmonomere. Aus diesem Grund werden beim Transport monomerer Acrylsäure Polymerisationsinhibitoren (Stabilisatoren) zugesetzt, um eine vorzeitige Polymerisation zu vermeiden.

Gebräuchliche Polymerisationsinhibitoren sind Phenothiazin (PTZ) oder phenolische Inhibitoren, wie Hydrochinon oder p-Methoxyphenol (Hydrochinonmonomethylether, MEHQ). Die phenolischen Inhibitoren entfalten ihre inhibierende Wirkung in Verbindung mit Sauerstoff, z. B. im Kontakt mit Luft. Für den Straßentransport wird Reinacrylsäure üblicherweise mit 200 ppm Polymerisationsinhibitor wie z. B. MEHQ stabilisiert.

Die WO 00/20369 empfiehlt zur Vermeidung einer radikalischen Polymerisation während des Transports von Acrylsäure den Zusatz eines phenolischen Polymerisationsinhibitors, wie p-Methoxyphenol, und eines Coinhibitors, insbesondere eines Mangankations. Der Coinhibitor kann z. B. mit einem Kationenaustauscher entfernt werden.

Die WO 03/095410 A1 offenbart ein Verfahren zur Herstellung einer wässrigen Alkaliacrylat-Lösung, bei dem man aus einem Gemisch Acrylsäure destillativ als Brüden abtrennt und den Brüden unmittelbar aus der Gasphase in eine wässrige Alkalilösung aufnimmt. Die wässrigen Alkaliacrylat-Lösungen sollen stark abgekühlt werden können, ohne dass eine Feststoffbildung erfolgt. Mit zunehmender Verdünnung der wässrigen Alkaliacrylat-Lösung sowie mit zunehmender Verringerung der möglichen Lagerungstemperatur ohne Feststoffabscheidung soll ein verringerter Bedarf an Lagerungsinhibitor für eine sichere Lagerung einhergehen.

Die US 5,130,471 beschreibt eine stabilisierte Acrylmonomer-Zusammensetzung, die ein Acrylmonomer, Phenothiazin und ein cyclisches Amin mit wenigstens einer Hydroxylgruppe umfasst.

Die EP-A 765 856 offenbart eine stabilisierte Monomerzusammensetzung, die neben Acrylsäure eine Kombination (i) eines Nitroxylradikals und/oder eines Hydroxylamins und (ii) einer diheterosubstituierten Benzolverbindung, wie p-Methoxyphenol, umfasst.

Obwohl MEHQ in Verbindung mit molekularem Sauerstoff äußerst wirksam monomere Acrylsäure stabilisiert, bildet es unter feuchten und/oder warmen Klimabedingungen farbige Zerfallsprodukte. Es ist bekannt, dass der Einsatz von MEHQ als Stabilisator zu Verfärbungen der Acrylsäure führt sowie Verfärbungen während der Lagerung von Superabsorbern und daraus hergestellter Erzeugnisse begünstigt. Diese Verfärbungen sind in der Regel unvermeidlich, da Superabsorber oder daraus hergestellte Erzeugnisse auf langen Transportwegen international versandt werden und mitunter längere Zeit, oft unter hoher Luftfeuchtigkeit, gelagert werden. Insbesondere beim Einsatz auf dem Hygienesektor sind verfärbte Produkte unerwünscht.

Als weiteres Problem tritt die Dimerenbildung der Acrylsäure auf. Bei der Dimerisierung wird ein Acrylsäuremolekül an die Doppelbindung eines weiteren Acrylsäuremoleküls addiert, so dass das Michael-Addukt β-Acryloxypropionsäure resultiert. Dimere Acrylsäure ist bereits nach einigen Stunden Standzeit nachweisbar, so dass bei längerer Stand- oder Transportzeit eine erhebliche Dimerenbildung auftritt. Die Diacrylsäurebildung wird durch hohe Temperatur und durch Anwesenheit von Wasser begünstigt.

Dimere Acryisäue beeinträchtigt zum Einen die Polymerisation der Acrylsäure. Ferner kann einpolymerisierte dimere Acrylsäure bei erhöhter Temperatur rückspalten. Dies äußert sich in einem hohen Restmonomergehalt der Polymerisate und führt zu Emissionen und Geruchsbelästigung.

Zur Begrenzung der Diacrylsäurebildung sollte Reinacrylsäure daher möglichst wasserfrei und bei möglichst tiefer Temperatur gelagert und/oder transportiert werden.

Die DE 10219089 empfiehlt zur Unterdrückung unerwünschter Diacrylsäurebildung, dass die Reinacrylsäure während der gesamten Dauer des Transportes und/oder der Lagerung teilkristallin vorliegt.

Acrylsäure hat einen Schmelzpunkt von 14 °C. Sie kann bei Temperaturen von 14 °C oder darunter in den festen Zustand übergehen. Das Auftauen von kristallisierter Reinacrylsäure erfordert äußerste Vorsicht, weil die Reinacrylsäure beim Kristallisieren an Polymerisationsinhibitor örtlich verarmt und entstabilisierte Acrylsäure unter großer Wärmeentwicklung explosionsartig polymerisieren kann. Die zum Auftauen eingesetzte externe Wärmequelle darf aus Sicherheitsgründen kein zu hohes Temperaturniveau aufweisen, weshalb das Auftauen einen vergleichsweise langen Zeitraum erfordert.

In der Praxis ist es daher von großer Bedeutung, das Gefrieren von Acrylsäure während des Transportes und/oder der Lagerung zu vermeiden. Acrylsäure muss daher in beheizten und/oder isolierten Behältern oder Rohrleitungen transportiert werden. Alternativ kann man die Acrylsäure mit ausreichend hoher Temperatur (wegen der Explosionsgefahr allerdings bis maximal etwa 30 °C) verladen, so dass bis zur Ankunft am Bestimmungsort eine Temperatur von 15 °C nicht unterschritten wird. Höhere Temperaturen begünstigen aber die Bildung von dimerer Acrylsäure.

Der Erfindung liegt die Aufgabe zugrunde, ein Verfahren zum sicheren Transport einer Monomer-Zusammensetzung in einem Verkehrsmittel oder einer Rohrleitung anzugeben, das die obigen Probleme minimiert oder überwindet. Der Erfindung liegt insbesondere die Aufgabe zugrunde, ein Transportverfahren anzugeben, bei dem die zum sicheren Transport erforderliche Menge an Polymerisationsinhibitor, wie p-Methoxyphenol, verringert werden kann.

Die Aufgabe wird gelöst durch ein Verfahren zum Transport einer Monomer-Zusammensetzung, dadurch gekennzeichnet, dass man eine Zusammensetzung, die eine wässrige Lösung von teilweise oder vollständig neutralisierter Acrylsäure oder überneutralisierter Acrylsäure umfasst, in ein Verkehrsmittel verbringt oder durch eine Rohrleitung schickt, wobei die Monomer-Zusammensetzung einen Polymerisationsinhibitor in Form von p-Methoxyphenol enthält und die Monomer-Zusammensetzung von anderen Polymerisationsinhibitoren im Wesentlichen frei ist, wobei der Gehalt der Monomer-Zusammensetzung an p-Methoxyphenol in ppm, bezogen auf Acrylsäure, gleich $\chi$ oder weniger ist, wobei $\chi$ gegeben ist durch die Gleichung:

$$\chi = 200 = 1{,}5 \cdot DN,$$

worin DN für den Neutralisationsgrad der Acrylsäure in Prozent steht.

Das erfindungsgemäße Verfahren zeichnet sich durch eine erhöhte Sicherheit beim Transport von Acrylsäuremonomeren, eine verbesserte Qualität der resultierenden Produkte, sowie eine hohe Wirtschaftlichkeit aus.

Durch den Einsatz des erfindungsgemäßen Verfahrens ist ein sicherer Transport der hochreaktiven Acrylsäure gewährleistet. Das Gefährdungspotential im Schadensfall durch vorzeitige Polymerisation unter extremer Hitzeentwicklung, wie es bei Reinacrylsäure vorliegt, wird durch das erfindungsgemäße Verfahren vollständig ausgeschlossen.

Die Einsatzmenge von Polymerisationsinhibitoren kann verringert werden. Eine aufwändige Entfernung von. Polymerisationsinhibitoren z. B. durch Behandlung mit Aktivkohle unmittelbar vor der Polymerisation kann entfallen. Ebenso bewirkt die verringerte Einsatzmenge von Polymerisationsinhibitoren eine dauerhafte Stabilität der hergestellten Produkte gegenüber vom Inhibitor herrührenden Verfärbungen.

Durch die Bereitstellung der wässrigen Lösung von teilweise neutralisierter Acrylsäure entfallen die Schritte des Auflösens bzw. Neutralisierens unmittelbar vor der Polymerisation am Polymerisationsstandort.

Ein weiterer wichtiger Vorteil des erfindungsgemäßen Verfahrens ist die Minimierung der Bildung dimerer Acrylsäure. Die Bildung dimerer Acrylsäure ist vom pH-Wert und somit vom Neutralisationsgrad abhängig. In teilweise oder vollständig neutralisierter Acrylsäure oder überneutralisierter Acrylsäure ist die Bildung dimerer Acrylsäure unterdrückt. Eine Abtrennung dimerer Acrylsäure vor der Polymerisation kann unterbleiben.

Ein zusätzlicher Vorteil besteht darin, dass eine Temperierung von Behältern, Rohrleitungen und Pipelines unterbleiben kann, in denen die Monomer-Zusammensetzung geführt wird, da diese bei deutlich niedrigeren Temperaturen als reine Acrylsäure kristallisiert.

Das erfindungsgemäße Verfahren zeichnet sich aufgrund der hervorragenden Sicherheit und der daraus resultierenden Vereinfachungen im Prozess durch eine hohe Wirtschaftlichkeit aus.

Zu den Verkehrsmitteln, mit denen die Monomer-Zusammensetzung transportiert werden kann, zählen Verkehrsmittel für den Straßen-, Bahn oder Schiffstransport, insbesondere Lastkraftwagen, Kesselwaggons oder Containerschiffe. Sie können Tankeinrichtungen aufweisen, die fest mit dem Verkehrsmittel verbunden sind, oder mit Behältern beladen werden, die zur Aufnahme von Flüssigkeiten geeignet sind, z. B. Tankcontainer. Rohrleitungen sind insbesondere Pipelines oder andere Überland-Rohrleitungen. Zu den Rohrleitungen zählen auch temporäre Verbindungen zum Be- und Entladen von Verkehrsmitteln.

Im Allgemeinen beträgt die Verweilzeit der Monomer-Zusammensetzung im Verkehrsmittel oder der Rohrleitung wenigstens 1 Stunde, meist wenigstens 5 Stunden, z. B. wenigstens 10 Stunden. Beim Transport in einem Verkehrsmittel kann die Verweilzeit z. B. 6 Stunden bis 25 Tage, vielfach 12 bis 48 Stunden, meist 24 bis 36 Stunden, betragen. Beim Transport in einem Verkehrsmittel wird als "Verweilzeit" die Zeit vom Befüllen des Verkehrsmittels bzw. eines auf das Verkehrsmittel zu verladenden Behälters bis zum vollständigen Entleeren angesehen. Beim Transport in einer Rohrleitung wird als "Verweilzeit" die mittlere Verweilzeit angesehen, die sich rechnerisch aus dem Leervolumen der Rohrleitung (Länge mal Querschnittsfläche) und dem Durchsatz (Volumen pro Zeiteinheit) ergibt.

Die erhöhte Sicherheit des erfindungsgemäßen Verfahrens kommt besonders zum Tragen, wenn große zusammenhängende Volumina der Monomer-Zusammensetzung transportiert werden, z. B. ein Volumen von wenigstens 1 m³, vorzugsweise wenigstens 5 m³ oder wenigstens 20 m³. Wenn ein Tank oder Kessel eines Verkehrsmittels kompartimentiert ist, wird als "zusammenhängendes Volumen" das Volumen eines Kompartiments angesehen. Beim Transport in einer Rohrleitung wird als "zusammenhängendes Volumen" das Leervolumen der Rohrleitung (Länge mal Querschnittsfläche) angesehen.

Im Allgemeinen hält man die Monomer-Zusammensetzung während des Transports bei einer Temperatur im Bereich von -10 bis 25 °C, vorzugsweise -5 bis 15°C, insbesondere 0 bis 10 °C.

Als Neutralisationsmittel eignen sich insbesondere Alkalimetallhydroxide, Alkatimetallcarbonate oder Alkalimetallhydrogencarbonate. Die Acrylsäure liegt somit vorzugsweise ganz oder teilweise als Alkalimetallsalz vor, insbesondere ganz oder teilweise als Natrium- und/oder Kaliumsalz. Als weitere Neutralisationsmittel kommen Ammoniak und Amine, wie Triethanolamin, in Betracht.

Unter "überneutralisierter Acrylsäure" wird eine Acrylsäurelösung verstanden, die mit einer größeren Menge an Neutralisationsmittel versetzt ist, als zur vollständigen Neutralisation der Acrylsäure erforderlich ist. Der bevorzugte Neutralisationsgrad der Acrylsäure beträgt 20 bis 110 Mol-%, z. B. 20 bis 100 Mol-%, vorzugsweise 20 bis 80 Mol%, insbesondere 40 bis 75 Mol-%. In bestimmten Ausführungsformen beträgt der Neutralisationsgrad 100 bis 110 Mol-%.

Die Monomer-Zusammensetzung umfasst eine wässrige Lösung von teilweise oder vollständig neutralisierter Acrylsäure oder überneutralisierter Acrylsäure, d. h. ein homogenes Gemisch aus Acrylsäure/Acrylsäuresalzen, Wasser und gegebenenfalls überschüssigem Neutralisationsmittel, worin der molare Anteil von Wasser in der Regel mehr als 50 mol-% beträgt, bezogen auf die Summe der Stoffmengen von Acrylsäure/Acrylsäuresalzen, Wasser und gegebenenfalls überschüssigem Neutralisationsmittel. Die Zusammensetzung enthält vorzugsweise 16 bis 56 Gew.-%, insbesondere 24 bis 40 Gew.-%, am meisten bevorzugt 28 bis 36 Gew.-%, Acrylsäure/Acrylsäuresalze, berechnet als Acrylsäure, d. h. sowohl Acrylsäuresalze als auch freie Acrylsäure werden als Acrylsäure (mit einer molaren Masse von 72,06 g/mol) gerechnet.

Aus Sicherheitsgründen und aufgrund behördlicher Auflagen ist ein vollständiger Verzicht auf Polymerisationsinhibitoren meistens nicht wünschenswert. Daher enthält die Monomer-Zusammensetzung in der Regel wenigstens einen Polymerisationsinhibitor.

Als Polymerisationsinhibitor wird p-Methoxyphenol verwendet und die Acrylatmonomer-Zusammensetzung ist von anderen Polymerisationsinhibitoren im Wesentlichen frei. Bei Verwendung von p-Methoxyphenol enthält die Zusammensetzung vorzugsweise gelösten Sauerstoff und/oder befindet sich im Kontakt mit der Umgebungsluft oder einem sauerstoffhaltigen Gasraum.

Vorzugsweise beträgt der Gesamtgehalt der Monomer-Zusammensetzung an Polymerisationsinhibitor(en) weniger als 100 ppm, vorzugsweise weniger als 50 ppm, insbesondere weniger als 40 ppm, am meisten bevorzugt weniger als 20 ppm, bezogen auf Acrylsäure.

Der Gehalt der Monomer-Zusammensetzung an p-Methoxyphenol in ppm, bezogen auf Acrylsäure ist gleich $\chi$ oder weniger, wobei $\chi$ gegeben ist durch die Gleichung:

$$\chi = 200 - 1{,}5 \cdot DN,$$

vorzugsweise

$$\chi = 200 - 1{,}8 \cdot DN,$$

worin DN für den Neutralisationsgrad der Acrylsäure in Mol-Prozent steht und DN im Bereich von 20 bis 110 Mol-% liegt.

Die Monomer-Zusammensetzung enthält in der Regel weniger als 100 ppm, insbesondere weniger als 20 ppm und speziell weniger als 10 ppm an solchen Verunreinigungen, die die Polymerisation der Acrylsäure nachteilig beeinflussen. Der Gehalt an aromatischen Aldehyden wie Benzaldehyd und Furfural liegt vorzugsweise unter 25 ppm und wird insbesondere 15 ppm nicht überschreiten. Der Gehalt an Prozessinhibitoren wie Phenothiazin liegt ebenfalls liegt vorzugsweise unter 10 ppm und wird insbesondere 5 ppm oder 2 ppm nicht überschreiten.

Vorzugsweise sind die folgenden Verunreinigungen in der Monomer-Zusammensetzung maximal in der angegebenen Konzentration enthalten:

| Dimere Acrylsäure | 1200 ppm |
|---|---|
| Acrolein | 50 ppm |

(fortgesetzt)

| Allylalkohol | 50 ppm |
|---|---|
| Allylacrylat | 20 ppm |
| Protoanemonin | 50 ppm |
| Propionsäure | 300 ppm |
| Essigsäure | 1000 ppm |
| Furfural | 22 ppm |
| Benzaldehyd | 1 ppm |
| Schwermetalle | 5 ppm (als Pb gerechnet) |
| Eisen | 2 ppm |
| Phenothiazin | 1 ppm |

Alle ppm Angaben sind Gew.-ppm, bezogen auf Acrylsäure.

Die Monomer-Zusammensetzung kann durch vollständige oder teilweise Neutralisation von Reinacrylsäure mit geeigneten Neutralisationsmitteln erhalten werden.

Im Allgemeinen stellt man Acrylsäure durch katalytische Gasphasenoxidation von $C_3$-Kohlenwasserstoffen wie Propan oder Propen und deren Gemische mit Sauerstoff her (zur Herstellung von Acrylsäure aus Propen siehe z.B. Ullmanns Enzyclopedia of Ind. Chem. 5th ed. on CD-ROM, "Acrylic acid and derivatives, 1.3.1. Propenoxidation", Wiley-VCH Weinheim 1997; K. Weisärmel, H.-J. Arpe "Industrielle Org. Chem., 4.Aufl., VCH Verlagsgesellschaft, Weinheim 1994, S. 315-17 sowie DE-A 29 43 707, DE-C 12 05 502, EP-A 117 146 EP-A 293 224 GB 1,450,986; zur Herstellung der Acrylsäure aus Propan siehe z.B. WO 99/20590 und der WO 00/53555).

Das bei der Oxidation von $C_3$-Kohlenwasserstoffen entstehende gasförmige Reaktionsgemische enthält als kondensierbare Komponenten neben einer Hauptmenge Acrylsäure in der Regel gesättigte Carbonsäuren wie Essigsäure und Propionsäure, eine Reihe von aromatischen Aldehyden wie Furfurale und Benzaldehyd, gegebenenfalls aliphatische Aldehyde wie Formaldehyd, Acrolein sowie gegebenenfalls Acetaldehyd und Propionaldehyd, Protoanemonin sowie diverse ungesättigte oder aromatische Carbonsäuren und deren Anhydride, z.B. Benzoesäure, Maleinsäure, Maleinsäureanhydrid und Phthalsäureanhydrid.

Zur Gewinnung der Acrylsäure aus dem Reaktionsgas sind zahlreiche Verfahren bekannt. Beispielsweise kann man eine Abtrennung der Acrylsäure aus dem heißen Reaktionsgas durch Aufnahme in ein geeignetes Absorptionsmittel, z.B. durch Gegenstromabsorption mit einem hochsiedenden Lösungsmittel, beispielsweise einem Gemisch aus Diphenylether und Diphenyl (siehe DE-A 21 36 396, DE-A 43 08 087 sowie Ullmanns Enzyclopedia of Ind. Chem. 5th ed. on CD-ROM, loc. cit.) oder durch Absorption in Wasser (siehe z.B. EP-A 511 111 und dort zitierte Literatur) erreichen und zur Gewinnung der Acrylsäure anschließend das Absorbtionsmittel entfernen, beispielsweise über destillative Trennverfahren.

In anderen Verfahren werden alle kondensierbaren Komponenten des Reaktionsgases, d.h. Acrylsäure, das Reaktionswasser sowie die oben erwähnten Verunreinigungen, weitgehend vollständig kondensiert (sog. Totalkondensat). Die hierbei erhaltene wasserhaltige Acrylsäure wird anschließend über Destillation mit azeotropen Schleppern (siehe beispielsweise DE-A 34 29 391 und JP-A 1124766), durch Extraktionsverfahren mit organischen Lösungsmitteln (siehe z.B. DE-A 21 64 767, JP-A 58140039; US 3,553,261, US 4,219,389, GB 1,427,223, US 3,962,074 und DE 23 23 328) vom Wasser weitgehend befreit.

Durch die oben genannten Verfahren gewinnt man Acrylsäure-Rohprodukte, die als Rohacrylsäure bezeichnet werden.

Die weitere Aufreinigung der Rohacrylsäure kann durch Destillation erfolgen. Die Destillation von Acrylsäure ist jedoch nicht unproblematisch, da sie bei thermischer Belastung sehr leicht polymerisiert. Daher müssen der Acrylsäure während der Destillation Prozess-Polymerisationsinhibitoren zugesetzt werden. Die als Destillat anfallende Acrylsäure wird dann mit einem Polymerisationsinhibitor für den Transport und/oder die Lagerung, z.B. Hydrochinonmonomethylether (MEHQ), versetzt.

Als Alternative zur Destillation wurde im Stand der Technik verschiedentlich auch die Kristallisation der Acrylsäure vorgeschlagen, beispielsweise in US 4,493,719, EP-A 616 998, EP-A 648 520, EP-A 715 870, EP 776 875, WO 98/25889 und WO 01/77056. Zur Gewinnung der aufgereinigten Acrylsäure wird das Kristallisat aufgeschmolzen. Aufgrund der hohen Polymerisationsneigung der dabei erhaltenen Acrylsäureschmelze müssen zu diesem Zeitpunkt Polymerisationsinhibitoren wie MEHQ zugesetzt werden, was die oben genannten Nachteile zur Folge hat.

In besonders zweckmäßiger Weise erhält man eine für die Herstellung von Acrylsäurepolymeren, insbesondere für die Herstellung von Acrylsäure basierten Superabsorbern geeignete Acrylsäure, wenn man Rohacrylsäure in an sich bekannter Weise kristallisiert und die kristallisierte Acrylsäure anstelle eines Aufschmelzvorgangs direkt in einer wässrigen Alkali-Lösung, insbesondere einer wässrigen Alkalimetallhydroxid-Lösung, löst.

Zweckmäßigerweise erhält man die Monomer-Zusammensetzung daher nach dem Verfahren der DE 102 21 202. Das Verfahren ist dadurch gekennzeichnet, dass man

i) eine Rohacrylsäureschmelze einer ein- oder mehrstufigen Kristallisation unterwirft, wobei man kristalline Acrylsäure und eine an Verunreinigungen angereicherte Acrylsäure-haltige Restschmelze erhält,

ii) die Restschmelze von der kristallinen Acrylsäure weitgehend oder vollständig abtrennt, und

iii) die kristalline Acrylsäure in einer zur Lösung der Acrylsäure ausreichenden Menge einer wässrigen Alkali-Lösung aufnimmt, wobei man eine teilweise oder vollständig neutralisierte Acrylsäure-Lösung erhält.

Die Durchführung der Kristallisation der Rohacrylsäure in Schritt i) erfolgt in an sich bekannter Weise. Üblicherweise überführt man die Rohacrylsäure in einen Kristallisator und kristallisiert unter Kühlen einen Teil der Acrylsäure aus. Diese wird nach üblichen Verfahren weitgehend oder vollständig von der Mutterlauge, d.h. der an Verunreinigungen angereicherten Restschmelze, abgetrennt. Gegebenenfalls kann man die so erhaltene kristalline Acrylsäure aufschmelzen und einer oder mehreren, z.B. 2, 3, 4, 5 oder 6 weiteren, aufeinanderfolgenden Kristallisationsstufen zuführen, bis der gewünschte Reinheitsgrad erreicht ist. Vorzugsweise arbeitet man dabei nach dem Gegenstromprinzip, d.h. die Mutterlauge der jeweiligen Kristallisationsstufe wird der jeweils vorangehenden Kristallisationsstufe zugeführt. Sofern man die Kristallisation als mehrstufige Kristallisation durchführt, kann man beim Aufschmelzen des Acrylsäurekristallisats geringe Mengen eines Stabilisators, vorzugsweise eines Hydrochinons oder eines Hydrochinonmonoalkylethers wie Hydrochinonmonomethylether zusetzen. Die Menge liegt dann in der Regel im Bereich von 1 bis 200 ppm, und insbesondere im Bereich von 5 bis 100 ppm, bezogen auf das Kristallisat. Ein Zusatz ist jedoch grundsätzlich nur dann in geringen Mengen erforderlich, wenn ein Aufschmelzen der Acrylsäure vorgenommen wird. D.h. im Anschluss an die letzte Kristallisationsstufe wird man in der Regel keinen oder nur geringe Mengen weiteren Stabilisator zusetzen und das Kristallisat auflösen. In der Regel führt man die Kristallisation in der jeweiligen Kristallisationsstufe so weit, dass wenigstens 10 Gew.-% und vorzugsweise wenigstens 20 Gew.-% der in der Rohacrylsäure enthaltenen Acrylsäure auskristallisiert sind. In der Regel wird man nicht mehr als 90 Gew.-%, vorzugsweise nicht mehr als 80 Gew.-% und insbesondere nicht mehr als 70 Gew.-% der in der jeweiligen Kristallisationsstufe eingesetzten Acrylsäure auskristallisieren, um eine hinreichende Reinigungswirkung zu erzielen.

In einer besonders bevorzugten Ausführungsform erfolgt die Kristallisation in Schritt i) als einstufige Kristallisation, d.h. die Kristallisation wird bis zu dem gewünschten Kristallisationsgrad geführt (Schritt i)), die Restschmelze, im Folgenden auch Mutterlauge wird von der kristallinen Acrylsäure abgetrennt (Schritt ii)) und die kristalline Acrylsäure in der wässrigen Alkali-Lösung aufgenommen ((Schritt iii)).

Die Abtrennung der Restschmelze von der kristallinen Acrylsäurephase erfolgt in an sich bekannter Weise nach üblichen Methoden zur Trennung flüssiger und fester Phasen. Dabei ist es nicht erforderlich, die Restschmelze vollständig von der kristallinen Phase zu trennen. Häufig enthält die in Schritt ii) abgetrennte Acrylsäure noch bis zu 10 Gew.-% Mutterlauge, z.B. 1 bis 10 Gew.-%, bezogen auf die insgesamt abgetrennte Acrylsäure. In der Regel führt man dann vor dem Auflösen der Acrylsäure in Schritt iii) einen der nachfolgend beschriebenen Reinigungsschritte durch.

Das Auflösen der kristallinen Acrylsäure in Schritt iii) erfolgt durch Behandeln der kristallinen Acrylsäure mit einer ausreichenden Menge einer wässrigen Alkali-Lösung. Unter wässrigen Alkalilösungen, versteht man Lösungen basischer, üblicherweise anorganischer Substanzen in Wasser, die Acrylsäure neutralisieren und die die Verwendung der Acrylsäure in der Polymerisation nicht nachteilig beeinflussen. Hierzu gehören Alkalimetallbasen wie Alkalimetallcarbonate, Alkalimetallhydrogencarbonate und Alkalimetallhydroxide, wobei letztere bevorzugt sind. Vorzugsweise setzt man in Schritt iii) eine wässrige Natronlauge oder eine Kalilauge ein. Die Konzentration an Alkali in diesen Lösungen wird in der Regel wenigstens 10 Gew.% betragen und liegt vorzugsweise im Bereich von 20 bis 60 Gew.%, insbesondere im Bereich von 20 bis 50 Gew.-%.

Die Monomer-Zusammensetzung ist zur Herstellung von Polymeren auf Basis von Acrylsäure besonders geeignet. Die Herstellung von SAP auf Basis von Acrylsäure erfolgt bekanntermaßen durch radikalische Polymerisation wässriger Monomerlösungen, die im Wesentlichen Acrylsäure und/oder Acrylsäuresalze als polymerisierbare Monomere enthalten. Die Polymerisation erfolgt vorzugsweise als Lösungs- bzw. Gel-Polymerisation in homogener wässriger Phase oder als Suspensionspolymerisation, wobei die wässrige Monomerlösung die disperse Phase bildet. Die bei der Polymerisation anfallenden wasserhaltigen Polymergele werden, gegebenenfalls nach einer Grobzerkleinerung, getrocknet und gegebenenfalls gemahlen. Die so erhaltenen partikulären Polymere werden in der Regel anschließend oberflächennachvernetzt.

Die Erfindung findet z.B. Anwendung in einem Verfahren zum Herstellen von wasserabsorbierenden Harzen, bei dem man

a) Acrylsäure herstellt und unmittelbar in eine wässrige Lösung von teilweise oder vollständig neutralisierter Acrylsäure oder überneutralisierter Acrylsäure überführt,
b) die so erhaltene Monomer-Zusammensetzung in einem Verkehrsmittel oder durch eine Rohrleitung transportiert,
c) gegebenenfalls den Neutralisationsgrad der Monomer-Zusammensetzung einstellt und gegebenenfalls weitere Monomere zugibt und eine Lösungs- oder Gelpolymerisation durchführt.

Unmittelbar vor der Polymerisation kann man gegebenenfalls den Neutralisationsgrad der Monomer-Zusammensetzung auf einen Wert einstellen, der für die Polymerisation erforderlich ist. Der Neutralisationsgrad für die Polymerisation beträgt z. B. 65 bis 75 Mol-% oder 40 bis 50 Mol-%. Wenn die transportierte Monomer-Zusammensetzung einen Neutralisationsgrad von beispielsweise 100 Mol-% aufweist, kann man durch Zugabe von Acrylsäure einen gewünschten niedrigeren Neutralisationsgrad einstellen,

Wie ausgeführt, bildet sich beim erfindungsgemäßen Transport dimere Acrylsäure nur in geringem Umfang. Dies ist von Vorteil, wenn das nach der Polymerisation erhaltene Polymer im Zuge seiner Aufarbeitung und/oder Weiterverarbeitung einem Hochtemperaturschritt, vorzugsweise bei einer Temperatur von wenigstens 150°C, z. B. 160 bis 190°C, unterzogen wird. Bei dieser Temperatur spaltet einpolymerisierte Dimeracrylsäure wieder ein Molekül Acrylsäure ab, weiche den Restmonomergehalt des fertigen Polymers erhöht.

Der Hochtemperaturschritt ist z. B. eine Oberflächennachvernetzung.

Vorzugsweise führt man die Polymerisation unter weitgehendem oder vollständigem Ausschluss von Sauerstoff durch. Vorzugsweise arbeitet man daher unter einer Inertgasatmosphäre. Als Inertgas wird insbesondere Stickstoff oder Wasserdampf eingesetzt. Insbesondere hat es sich bewährt, die zu polymerisierende wässrige Monomerlösung bzw. das monomerhaltige wässrige Polymerisationsmedium vor und/oder während der Polymerisation mit Inertgas zu spülen.

Die Polymerisation erfolgt in der Regel im Temperaturbereich von 0 °C bis 150 °C, vorzugsweise im Bereich von 10 °C und 100 °C, und kann sowohl bei Normaldruck als auch unter erhöhtem oder erniedrigtem Druck durchgeführt werden.

Bezogen auf ihr Gesamtgewicht enthält die zu polymerisierende Monomer-Zusammensetzung in der Regel:

- 50 bis 99,99 Gew.-%, vorzugsweise 70 bis 99,9 Gew.-% und insbesondere 80 bis 99,8 Gew.-% Acrylsäure(salze) als Monomer A,

- 0 bis 49,99 Gew.-%, insbesondere 0 bis 29,9 Gew.-% und insbesondere 0 bis 19,8 Gew.-% eines oder mehrerer mit Acrylsäure copolymerisierbarer, monoethylenisch ungesättigter Monomere B und

- 0,01 bis 20 Gew.-%, insbesondere 0,1 bis 15 Gew.-% und insbesondere 0,2 bis 3 Gew.-% wenigstens einer vernetzend wirkenden Verbindung C.

Hier und im Folgenden sind alle Gewichtsanteile auf das Gesamtgewicht aller zu polymerisierenden Monomere bezogen, wobei Gewichtsangaben von Säuregruppentragenden Monomeren, die auch als Salze vorliegen können, stets auf die Säureform bezogen sind.

Beispiele für geeignete Monomere B sind von Acrylsäure verschiedene Säuregruppentragende Monomere B1, z.B. monoethylenisch ungesättigte Mono- und Dicarbonsäuren mit vorzugsweise 4 bis 8 C-Atomen wie Methacrylsäure, Ethacrylsäure, α-Chlor-acrylsäure, Crotonsäure, Maleinsäure, Maleinsäureanhydrid, Itaconsäure, Citraconsäure, Mesaconsäure, Glutaconsäure, Aconitsäure und Fumarsäure; Halbester von monoethylenisch ungesättigten Dicarbonsäuren mit 4 bis 10 vorzugsweise 4 bis 6 C-Atomen, z. B. von Maleinsäure wie Maleinsäuremonomethylester; monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure und die Salze, insbesondere die Natrium-, Kalium- und Ammoniumsalze dieser Säuren.

Bevorzugte Monomere B1 sind Methacrylsäure, Vinylsulfonsäure, Styrolsulfonsäure, 2-Acrylamido-2-methylpropansulfonsäure oder Mischungen dieser Säuren. Der Anteil der Monomere B1 an der Gesamtmonomermenge macht, sofern erwünscht, vorzugsweise 0,1 bis 29,9 und insbesondere 0,5 bis 19,8 Gew.-%, bezogen auf die Gesamtmonomermenge aus.

Zur Optimierung von Eigenschaften der Polymerisate kann es sinnvoll sein, monoethylenisch ungesättigte Monomere B2 einzusetzen, die keine Säuregruppen tragen, aber mit Acrylsäure und ggf. den Monomeren B1 copolymerisierbar sind und nicht vernetzend wirken. Hierzu gehören beispielsweise monoethylenisch ungesättigte Nitrile wie Acrylnitril, Methacrylnitril, die Amide der vorgenannten monoethylenisch ungesättigten Carbonsäuren, z.B. Acrylamid, Methacrylamid, N-Vinylamide wie N-Vinylformamid, N-Vinylacetamid, N-Methylvinylacetamid, N-Vinylpyrrolidon und N-Vinylcaprolactam. Zu den Monomeren B2 zählen außerdem Vinylester gesättigte $C_1$-$C_4$-Carbonsäuren wie Vinylformiat, Vinylacetat und Vinylpropionat, Alkylvinylether mit mindestens 2 C-Atomen in der Alkylgruppe, z. B. Ethylvinylether oder Butylvinylether, Ester monoethylenisch ungesättigter $C_3$-$C_6$-Carbonsäuren, z. B. Ester aus einwertigen $C_1$-$C_{18}$-Alkoholen und Acrylsäure, Methacrylsäure oder Maleinsäure, Acrylsäure- und Methacrylsäureester von alkoxylierten einwertigen, gesättigten Alkoholen, z. B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen (Mn) der Polyalkylenglykole beispielsweise bis zu 2000 betragen können. Weiterhin geeignete Monomere B2 sind Styrol und alkylsubstituierte Styrole wie Ethylstyrol oder tert-Butylstyrol. Der Anteil der Monomere B2 an der Gesamtmonomermenge wird vorzugsweise 20 Gew.% nicht überschreiten und macht, sofern erwünscht, vorzugsweise 0,1 bis 20 Gew.% aus.

Als vernetzend wirkende Verbindungen C kommen solche Verbindungen in Betracht, die mindestens zwei, z. B. 2, 3, 4 oder 5 ethylenisch ungesättigte Doppelbindungen im Molekül aufweisen. Diese Verbindungen werden auch als Vernetzermonomere C1 bezeichnet. Beispiele für Verbindungen C1 sind N,N'-Methylenbisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Ethylenglykoldimethacrylat, Propylenglykoldiacrylat, Propylenglykoldimethacrylat, Butandioldiacrylat, Butandioldimethacrylat, Hexandioldiacrylat, Hexandioldimethacrylat, Diethylenglykoldiacrylat, Diethylenglykoldimethacrylat, Triethylenglykoldiacrylat, Triethylenglykoldimethacrylat, Dipropylenglykoldiacrylat, Dipropylenglykoldimethacrylat, Tripropylenglykoldiacrylat, Tripropylenglykoldimethacrylat, Allylmethacrylat, Diacrylate und Dimethacrylate von Blockcopolymerisaten aus Ethylenoxid und Propylenoxid, zwei-, drei-, vier- oder fünffach mit Acrylsäure oder Methacrylsäure veresterte mehrwertige Alkohole, wie Glycerin, Trimethylolpropan, Pentaerythrit oder Dipentaerythrit, Ester monoethylenisch ungesättigter Carbonsäuren mit ethylenisch ungesättigten Alkoholen wie Allylalkohol, Cyclohexenol und Dicyclopentenylalkohol, z. B. Allylacrylat und Allylmethacrylat, weiterhin Triallylamin, Dialkyldiallylammoniumhalogenide wie Dimethyldiallylammoniumchlorid und Diethyldiallylammoniumchlorid, Tetraallylethylendiamin, Divinylbenzol, Diallylphthalat, Polyethylenglykoldivinylether von Polyethylenglykolen eines Molekulargewichtes von 106 bis 4000, Trimethylolpropandiallylether, Butandioldivinylether, Pentaerythrittriallylether, Umsetzungsprodukte von 1 Mol Ethylenglykoldiglycidylether oder Polyethylenglykoldiglycidylether mit 2 Mol Pentaerythritoltriallylether oder Allylalkohol, und Divinylethylenharnstoff. Der Anteil der Monomere C1 an der zu polymerisierenden Monomermischung beträgt vorzugsweise 0,01 bis 5 Gew.% und insbesondere 0,2 bis 3 Gew.-%.

Als vernetzend wirkende Verbindungen C können ferner Verbindungen C2 mit funktionellen Gruppen fungieren, die mit wenigstens 2 Carboxylgruppen des Polymers unter Ausbildung einer kovalenten Bindung reagieren können (gegenüber Carboxylgruppen komplementär funktionelle Gruppen). Als Vernetzer C kommen auch vernetzend wirkende Monomere C3 in Betracht, die neben einer ethylenisch ungesättigten Doppelbindung wenigstens eine weitere gegenüber Carboxylgruppen komplementäre funktionelle Gruppe aufweisen. In Betracht kommen auch Polymere mit einer Vielzahl derartiger funktioneller Gruppen. Geeignete funktionelle Gruppen sind z.B. Hydroxyl-, Amino-, Epoxy- und Aziridingruppen, weiterhin Isocyanat-, Ester- und Amidogruppen sowie Alkyloxysilylgruppen. Zu den geeigneten Vernetzern dieses Typs zählen beispielsweise Aminoalkohole, wie Ethanolamin oder Triethanolamin, Di- und Polyole, wie 1,3-Butandiol, 1,4-Butandiol, Ethylenglykol, Diethylenglykol, Triethylenglykol, Tetraethylenglykol, Polyethylenglykol, Glycerin, Polyglycerin, Propylenglykol, Polypropylenglykol, Trimethylolpropan, Pentaerythrit, Polyvinylalkohol, Sorbit, Stärke, Blockcopolymerisate aus Ethylenoxid und Propylenoxid, Polyamine wie Ethylendiamin, Diethylentriamin, Triethylentetramin, Tetraethylenpentamin, Pentaethylenhexamin und Polyethylenimine sowie Polyamine mit Molmassen von jeweils bis zu 4000000, Ester wie Sorbitanfettsäureester, ethoxylierte Sorbitanfettsäureester, Polyglycidylether wie Ethylenglykoldiglycidylether, Polyethylenglykol-diglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanol-tris[3-(1-aziridinyl)-propionat], Diamide der Kohlensäure, wie 1,6-Hexamethylendiethylenharnstoff, Diphenylmethan-bis-4,4'-N,N'-diethylenharnstoff, Halogenepoxyverbindungen, wie Epichlorhydrin und a-Methylepifluorhydrin, Polyisocyanate, wie 2,4-Toluylendiisocyanat und Hexamethylendiisocyanat, Alkylencarbonate wie 1,3-Dioxolan-2-on und 4-Methyl-1,3-dioxolan-2-on, weiterhin Bisoxazoline und Oxazolidone, Polyamidoamine sowie deren Umsetzungsprodukte mit Epichlorhydrin, ferner polyquaternäre Amine, wie Kondensationsprodukte von Dimethylamin mit Epichlorhydrin, Homo- und Copolymere von Diallyldimethylammoniumchlorid sowie Homound Copolymerisate von Dimethylaminoethyl(meth)acrylat, die gegebenenfalls mit beispielsweise Methylchlorid quaterniert sind. Beispiele für Verbindungen C3 sind Hydroxyalkylacrylate und -methacrylate sowie Glycidylester der vorgenannten ethylenisch ungesättigten Carbonsäuren und ethylenisch ungesättigte Glycidylether.

Vorzugsweise umfassen die Monomere C wenigstens ein Monomer C1 in den obengenannten Mengen. Vorzugsweise erfolgt die Polymerisation in Abwesenheit von Verbindungen C2.

Geeignete Pfropfgrundlagen können natürlichen oder synthetischen Ursprungs sein. Hierzu zählen Stärken, d. h. native Stärken aus der Gruppe der Maisstärke, Kartoffelstärke, Weizenstärke, Reisstärke, Tapiokastärke, Sorghunstärke, Maniokstärke, Erbsenstärke oder deren Mischungen, modifizierte Stärken, Stärkeabbauprodukte, z. B. oxidativ, enzymatisch oder hydrolytisch abgebaute Stärken, Dextrine, z. B. Röstdextrine sowie niedere Oligo- und Polysaccharide, z. B. Cyclodextrine mit 4 bis 8 Ringgliedern. Als Oligo- und Polysaccharide kommen weiterhin Cellulose, Stärke- und Cellulosederivate in Betracht. Ferner eignen sich Polyvinylalkohole, Homo- und Copolymere des N-Vinylpyrrolidons, Polyamine, Polyamide, hydrophile Polyester oder Polyalkylenoxide, insbesondere Polyethylenoxid und Polypropylenoxid. Geeignete Polyalkylenoxide weisen die allgemeine Formel I auf,

$$R^1\text{-O-(CH}_2\text{-CHX-O)}_n\text{-R}^2$$

worin $R^1$, $R^2$ unabhängig voneinander für Wasserstoff; $C_1$-$C_4$-Alkyl; $C_2$-$C_6$-Alkenyl, insbesondere Phenyl; oder (Meth) acryl stehen; X für Wasserstoff oder Methyl und n für eine ganze Zahl von 1 bis 1000, insbesondere 10 bis 400 steht.

Als Polymerisationsreaktoren kommen die zur Herstellung üblichen Reaktoren, insbesondere Bandreaktoren, Extruder und Kneter, in Betracht (siehe "Modern Superabsorbent Polymer Technology", Kapitel 3.2.3). Die Polymerisate werden besonders bevorzugt nach einem kontinuierlichen oder diskontinuierlichen Knetverfahren oder einem kontinuierlichen Bandpolymerisationsverfahren hergestellt.

Als Initiatoren kommen grundsätzlich alle Verbindungen in Betracht, die beim Erwärmen auf Polymerisationstemperatur oder aufgrund einer Redoxreaktion unter Bildung von Radikalen zerfallen. Die Polymerisation kann auch durch Einwirkung energiereicher Strahlung, z. B. UV-Strahlung, in Gegenwart von Photoinitiatoren ausgelöst werden. Auch eine Initiierung der Polymerisation durch Einwirkung von Elektronenstrahlen auf die polymerisierbare, wässrige Mischung ist möglich.

Geeignete Initiatoren sind beispielsweise Peroxoverbindungen wie organische Peroxide, organische Hydroperoxide, Wasserstoffperoxid, Persulfate, Perborate, Azoverbindungen und die sogenannten Redoxkatalysatoren. Bevorzugt werden wasserlösliche Initiatoren. In manchen Fällen ist es vorteilhaft, Mischungen verschiedener Polymerisationsinitiatoren zu verwenden, z. B. Mischungen aus Wasserstoffperoxid und Natrium-oder Kaliumperoxodisulfat. Geeignete organische Peroxide sind beispielsweise Acetylacetonperoxid, Methylethylketonperoxid, tert-Butylhydroperoxid, Cumolhydroperoxid, tert-Amylperpivalat, tert-Butylperpivalat, tert-Butylperneohexanoat, tert-Butylperisobutyrat, tert-Butyl-per-2-ethylhexanoat, tert-Butylperisononanoat, tert-Butylpermaleat, tert-Butylperbenzoat, Di-(2-ethylhexyl)peroxidicarbonat, Dicyclohexylperoxidicarbonat, Di-(4-tert.-butylcyclohexyl)peroxidicarbonat, Dimyristilperoxidicarbonat, Diacetylperoxidicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z. B. 2,2'-Azo-bis-(2-amidinopropan)dihydrochlorid, 2,2'-Azobis-(N,N'-dimethylen)isobutyramidin-dihydrochlorid, 2-(Carbamoylazo)isobutyronitril, 2,2'-Azo-bis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z.B. in Mengen von 0,01 bis 5, vorzugsweise 0,05 bis 2,0 Gew.%, meist 0,05 bis 0,30 Gew.-%, bezogen auf die zu polymerisierenden Monomeren.

Redoxinitiatoren sind bevorzugt. Sie enthalten als oxidierende Komponente mindestens eine der oben angegebenen Peroxoverbindungen und als reduzierende Komponente beispielsweise Ascorbinsäure, Glukose, Sorbose, Ammonium- oder Alkalimetallsulfit, -hydrogensulfit, -thiosulfat, -hyposulfit, -pyrosulfit oder -sulfid, Metallsalze, wie Eisen (II)-ionen oder Natriumhydroxymethylsulfoxylat. Vorzugsweise verwendet man als reduzierende Komponente des Redoxkatalysators Ascorbinsäure oder Natriumsulfit. Als reduzierende Komponente ist auch ein Gemisch aus dem Natriumsalz der 2-Hydroxy-2-sulfinatoessigsäure, dem Dinatriumsalz der 2-Hydroxy-2-sulfonatoessigsäure und Natriumbisulfit bevorzugt. Derartige Gemische sind als Brüggolite® FF6 und Brüggolite® FF7 (Brüggemann Chemicals; Heilbronn; DE) erhältlich. Bezogen auf die bei der Polymerisation eingesetzte Menge an Monomeren verwendet man beispielsweise $3\times10^{-6}$ bis 1 Mol% der reduzierenden Komponente des Redoxkatalysatorsystems und 0,001 bis 5,0 Mol% der oxidierenden Komponente des Redoxkatalysators.

Wenn man die Polymerisation durch Einwirkung energiereicher Strahlung auslöst, verwendet man üblicherweise als Initiator sogenannte Photoinitiatoren.

Der Feuchtigkeitsgehalt des wasserhaltigen Polymergels liegt in der Regel im Bereich 20 bis 80 Gew.-%. Das wasserhaltige Polymergel wird dann in an sich bekannter Weise in ein partikuläres Polymer überführt und anschließend oberflächennachvernetzt.

Hierzu wird das bei der Polymerisation anfallende wasserhaltige Polymergel in der Regel zunächst nach bekannten Methoden zerkleinert. Die Grobzerkleinerung der wasserhaltigen Polymergel erfolgt mittels üblicher Reiss- und/oder Schneidwerkzeuge, z. B. durch die Wirkung einer Austragspumpe im Falle der Polymerisation in einem zylindrischen Reaktor oder durch eine Schneidwalze oder Schneidwalzenkombination im Falle der Bandpolymerisation. Eine weitere Zerkleinerung erfolgt in der Regel mit einem Gel Chopper. Im Fall der Polymerisation in einem Knetreaktor fällt unmittelbar ein trocknungsfähiges wassserhaltiges Polymergel an.

Das so erhaltene grob zerleinerte Polymergel wird anschließend bei erhöhter Temperatur, z. B. im Bereich von 80°C bis 250°C und insbesondere im Bereich von 100 °C bis 180 °C, nach bekannten Verfahren getrocknet (siehe "Modern Superabsorbent Polymer Technology" Kapitel 3.2.5). Hierbei erhält man partikuläre Polymere in Form von Pulvern oder Granulaten, die gegebenenfalls zur Einstellung der Partikelgröße noch mehreren Mahl- und Siebvorgängen unterworfen werden (siehe "Modern Superabsorbent Polymer Technology" Kapitel 3.2.6 und 3.2.7).

Vorzugsweise umfasst das Verfahren eine Oberflächennachvernetzung. Die Oberflächennachvernetzung erfolgt in an sich bekannter Weise mit getrockneten, vorzugsweise gemahlenen und abgesiebten Polymerpartikeln. Zur Oberflächenvernetzung werden Verbindungen mit funktionellen Gruppen eingesetzt, die mit wenigstens zwei Carboxylgruppen der Polymere unter Vernetzung reagieren können (Nachvernetzungsmittel). Die funktionellen Gruppen können im Nachvernetzungsmittel in latenter Form vorliegen, d.h. sie werden erst unter den Reaktionsbedingungen der Oberflächennachvernetzung freigesetzt. Geeignete funktionelle Gruppen in Nachvernetzungsmitteln sind Hydroxylgruppen, Glycidylgruppen, Alkoxysilylgruppen, Aziridingruppen, primäre und sekundäre Aminogruppen, N-Methylolgruppen (= N-Hydroxymethylgruppen, $N-CH_2-OH$-Gruppen), Oxazolidin-Gruppen, Harnstoff- und Thioharnstoffgruppen, gegebenenfalls reversibel blockierte Isocyanat-Gruppen sowie cyclische Carbonat-Gruppen wie in Ethylencarbonat. Zur Oberflächennachvernetzung werden die Nachvernetzungsmittel, vorzugsweise in Form einer wässrigen Lösung auf die Oberfläche der Polymerisat-Partikel aufgebracht. Die wässrige Lösung kann wassermischbare organische Lösungsmittel enthalten. Geeignete Lösungsmittel sind z.B. $C_1$-$C_4$-Alkohole wie Methanol, Ethanol, Isopropanol oder Ketone wie Aceton und Methylethylketon.

Geeignete Nachvernetzungsmittel sind beispielsweise:

- Di- oder Polyglycidylverbindungen wie Phosphonsäurediglycidylether oder Ethylenglykoldiglycidylether, Bischlorhydrinether von Polyalkylenglykolen,

- Alkoxysilylverbindungen,

- Polyaziridine, Aziridin-Einheiten enthaltende Verbindungen auf Basis von Polyethern oder substituierten Kohlenwasserstoffen, beispielsweise Bis-N-aziridinomethan,

- Polyamine oder Polyamidoamine sowie deren Umsetzungsprodukte mit Epichlorhydrin,

- Diole und Polyole, z.B. Ethylenglykol, 1,2-Propandiol, 1,4-Butandiol, Glycerin, Methyltriglykol, Trimethylolethan, Trimethylolpropan, Polyethylenglykole mit einem mittleren Molekulargewicht Mw von 200 - 10000, Di- und Polyglycerin, Pentaerythrit, Sorbit, die Oxethylate dieser Polyole sowie deren Ester mit Carbonsäuren oder mit Kohlensäure wie Ethylencarbonat oder Propylencarbonat,

- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,

- Di- und Poly-N-methylolverbindungen wie beispielsweise Methylenbis(N-methylol-methacrylamid) oder Melamin-Formaldehyd-Harze,

- Verbindungen mit zwei oder mehr blockierten Isocyanat-Gruppen wie beispielsweise Trimethylhexamethylendiisocyanat blockiert mit 2,2,3,6-Tetramethylpiperidinon-4.

Bei Bedarf können saure Katalysatoren wie p-Toluolsulfonsäure, Phosphorsäure, Borsäure oder Ammoniumdihydrogenphosphat zugesetzt werden.

Das Aufbringen der Vernetzer-Lösung erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt nachfolgen, bevorzugt in einem nachgeschalteten Trockner, bei einer Temperatur zwischen 80 und 230 °C, bevorzugt zwischen 100 und 160 °C oder 180 und 200 °C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können. Die Trocknung kann aber auch im Mischer selbst erfolgen; durch Beheizung des Mantels oder Einblasen eines vorgewärmten Trägergases.

Die erhaltenen SAP sind insbesondere zur Herstellung von Hygieneartikeln geeignet. Der Aufbau und die Form von Hygieneartikeln, insbesondere Windeln, Binden und Inkontinenzeinlagen und -hosen für Erwachsene, ist allgemein bekannt und beispielsweise in der EP-A-0 316 518, der EP-A-0 202 127, der DE 19737434, der WO 00/65084, der WO 00/65348 und der WO 00/35502, beschrieben.

Typische Hygieneartikel in Form von Windeln, Binden und Inkontinenzeinlagen und - hosen umfassen:

(A) eine obere flüssigkeitsdurchlässige Abdeckung

(B) eine untere flüssigkeitsundurchlässige Schicht

(C) einen zwischen (A) und (B) befindlichen Kern, enthaltend

(C1) 10 - 100 Gew.-% wasserabsorbierendes Harz

(C2) 0 - 90 Gew.-% hydrophiles Fasermaterial

(D) gegebenenfalls eine sich unmittelbar oberhalb und unterhalb des Kerns (C) sich befindende Tissueschicht und

(E) gegebenenfalls eine zwischen (A) und (C) sich befindende Aufnahmeschicht.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filmen von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen.

Die flüssigkeitsundurchlässige Schicht (B) besteht in der Regel aus einer Folie aus Polyethylen oder Polypropylen.

Der Kern (C) enthält neben dem wasserabsorbierenden Harz (C1) hydrophiles Fasermaterial (C2). Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 bis 200 $\mu$m, bevorzugt 10 bis 100 $\mu$m. Darüber hinaus haben die Fasern eine Mindestlänge von 2 mm.

Der Anteil des hydrophilen Fasermaterials bezogen auf die Gesamtmenge des Kerns beträgt bevorzugt 20 bis 80 Gew.%, besonders bevorzugt 40 bis 70 Gew.-%.

Die Erfindung wird durch die folgenden Beispiele näher veranschaulicht.

Beispiel 1: Lagerstabilität von wässriger Acrylsäure und neutralisierter Acrylsäure

Zur Herstellung der unneutralisierten Acrylsäurelösung legte man 1071,3 g VE-Wasser vor und löste 428,7 g (5,95 mol) Acrylsäure (stabilisiert mit 50ppm MEHQ) darin. Zur Herstellung einer wässrigen Natriumacrylatlösung (100 % Neutralisationsgrad) legte man 595,4 g entmineralisiertem Wasser in einem 3L Plastikbecher vor und löste 476,0 g Natronlauge (50 %ig) darin. Über einen 500 ml Tropftrichter tropfte man 428,7 g (5,95 mol) Acrylsäure (stabilisiert mit 50ppm MEHQ) zu. Während der Dosierung kühlte man das Plastikbecherglas im Eisbad und wählte die Dosiergeschwindigkeit so, dass die Temperatur im Inneren des Plastikbecherglases 30°C nicht überschritt.

Aliquote (400 g) der so hergestellten wässrigen Acrylsäure- und Natriumacrylatlösung wurden in 500 mL Schraubdeckelgläser gefüllt. Der Deckel wurde locker angezogen, so dass im Falle einer Polymerisation Überdruck entweichen kann. Die Aliquote wurden bei 60°C gelagert und die Lagerstabilität mittels Viskositätsmessung überwacht.

Zur Durchführung der Viskositätsmessung wurden die Proben jeweils auf 22°C (+/-2°C) abgekühlt. Die Viskosität wurde mit einen Brookfield Viskosimeter (Model LVT, Spindel 1) gemessen.

Die Ergebnisse der Lagerversuche sind in den nachstehenden Tabellen zusammengefasst.

Lagerung bei 60 °C

| Tag d. Lagerung | Natriumacrylatlösung | wässrige Acrylsäure |
|---|---|---|
| | Viskosität [mPa*s] (Beobachtung) | |
| 0 | 7,5 | 3,0 |
| 2 | 6,5 | 3,0 |

(fortgesetzt)

| Tag d. Lagerung | Natriumacrylatlösung | wässrige Acrylsäure |
|---|---|---|
| | Viskosität [mPa*s] (Beobachtung) | |
| 3 | 5,0 | 2,0 |
| 4 | 6,5 | 2,5 |
| 7 | 6,5 | 2,5 |
| 8 | 6,5 | 2,5 |
| 9 | 9,0 | 4,5 (zieht Fäden) |
| 10 | 9,0 | 5,0 |
| 11 | 9,5 | 5,0 |
| 15 | 9,0 | 8,5 (anpolymerisiert - Polymer sichtbar) |
| 16 | 9,0 | 9 (anpolymerisierter Anteil ist größer geworden) |
| 17 | 9,0 | 13,0 |
| 18 | 9,0 | weitgehend geliert, nicht messbar |
| 21 | 9,0 | vollständig geliert |
| 22 | 9,0 | vollständig geliert |
| 23 | 9,0 | vollständig geliert |
| 24 | 9,0 | vollständig geliert |
| 30 | 9,0 | vollständig geliert |

Lagerung bei 60 °C (Wiederholungsversuch)

| Tag d. Lagerung | Natriumacrylatlösung | wässrige Acrylsäure |
|---|---|---|
| | visuelle Beurteilung | |
| 2 | unverändert | unverändert |
| 4 | unverändert | unverändert |
| 7 | unverändert | unverändert |
| 9 | unverändert | vollständig geliert |
| 10 | unverändert | vollständig geliert |
| 14 | unverändert | vollständig geliert |
| 17 | unverändert | vollständig geliert |
| 22 | unverändert | vollständig geliert |
| 25 | unverändert | vollständig geliert |

Lagerung bei 60 °C (2. Wiederholungsversuch)

| Tag d. Lagerung | Natriumacrylatlösung | wässrige Acrylsäure |
|---|---|---|
| | visuelle Beurteilung | |
| 2 | unverändert | unverändert |
| 4 | unverändert | unverändert |
| 7 | unverändert | unverändert |
| 9 | unverändert | unverändert |
| 10 | unverändert | vollständig geliert |
| 14 | unverändert | vollständig geliert |
| 17 | unverändert | vollständig geliert |
| 22 | unverändert | vollständig geliert |
| 25 | unverändert | vollständig geliert |

Beispiel 2: Bildung von β-Acryloxypropionsäure

Man lagerte Aliquote wässriger Acrylsäurelösung bzw. Natriumacrylatlösung 1 Woche bei 6 °C bzw. 23 °C und bestimmte dann den Gehalt an β-Acryloxypropionsäure durch HPLC (Säule: Waters Symmetry 150 x 3,9 mm; 25 °C; mobile Phase: 90 Vol.-% Phosphorsäure (0,1 Vol.-%)/10 Vol.% Acetonitril; Detektion bei 210 nm). Die Ergebnisse sind in der nachstehenden Tabelle zusammengefasst.

Lagerung bei 6 °C

| Neutralisationsgrad [%] | Feststoffgehalt [Gew.-%] | β-Acryloxypropionsäure [ppm] | |
|---|---|---|---|
| | | anfänglich | nach 1 Woche |
| 0 | 40 | 55 | 173 |
| 100 | 37 | 5 | 5 |

Lagerung bei 23 °C

| Neutralisationsgrad [%] | Feststoffgehalt [Gew.-%] | β-Acryloxypropionsäure [ppm] | |
|---|---|---|---|
| | | anfänglich | nach 1 Woche |
| 0 | 40 | 29 | 824 |
| 100 | 37 | 7 | 4 |

**Patentansprüche**

1.  Verfahren zum Transport einer Monomer-Zusammensetzung, **dadurch gekennzeichnet, dass** man eine Zusammensetzung, die eine wässrige Lösung von teilweise oder vollständig neutralisierter Acrylsäure oder überneutralisierter Acrylsäure umfasst, in ein Verkehrsmittel verbringt oder durch eine Rohrleitung schickt, wobei die Monomer-Zusammensetzung einen Polymerisationsinhibitor in Form von p-Methoxyphenol enthält und die Monomer-Zusammensetzung von anderen Polymerisationsinhibitoren im Wesentlichen frei ist, wobei der Gehalt der Monomer-Zusammensetzung an p-Methoxyphenol in ppm, bezogen auf Acrylsäure, gleich χ oder weniger ist, wobei χ gegeben ist durch die Gleichung:

$$\chi = 200 - 1{,}5 \cdot DN,$$

worin DN für den Neutralisationsgrad der Acrylsäure in Prozent steht

**2.** Verfahren nach Anspruch 1, **dadurch gekennzeichnet, dass** die Acrylsäure ganz oder teilweise als Alkalimetallsalz vorliegt.

**3.** Verfahren nach Anspruch 2, **dadurch gekennzeichnet, dass** die Acrylsäure ganz oder teilweise als Natrium- und/oder Kaliumsalz vorliegt.

**4.** Verfahren nach einem der vorhergehenden Ansprüche, **dadurch gekennzeichnet, dass** der Neutralisationsgrad der Acrylsäure 20 bis 110 Mol-%, vorzugsweise 20 bis 80 Mol-%, beträgt.

**5.** Verfahren nach einem der vorhergehenden Ansprüche, **dadurch gekennzeichnet, dass** die Zusammensetzung 16 bis 56 Gew.-%, vorzugsweise 24 bis 40 Gew.-%, Monomer, berechnet als Acrylsäure, enthält.

**6.** Verfahren nach einem der vorhergehenden Ansprüche, **dadurch gekennzeichnet, dass** die Verweilzeit der Monomer-Zusammensetzung im Verkehrsmittel oder der Rohrleitung wenigstens 1 Stunde beträgt.

**7.** Verfahren nach einem der vorhergehenden Ansprüche, **dadurch gekennzeichnet, dass** man die Monomer-Zusammensetzung als zusammenhängendes Volumen von wenigstens 1 m$^3$ transportiert.

**8.** Verfahren nach einem der vorhergehenden Ansprüche, wobei die Zusammensetzung gelösten Sauerstoff enthält und/oder sich im Kontakt mit der Umgebungsluft oder einem sauerstoffhaltigen Gasraum befindet.

**9.** Verfahren nach einem der vorhergehenden Ansprüche, **dadurch gekennzeichnet, dass** die Monomer-Zusammensetzung zur Herstellung wasserabsorbierender Harze geeignet ist.

**Claims**

**1.** A process for transporting a monomer composition, which comprises transferring a composition comprising an aqueous solution of partly or fully neutralized acrylic acid or overneutralized acrylic acid to a mode of transport or sending it through a pipeline, wherein the monomer composition comprises a polymerization inhibitor in the form of p-methoxyphenol and the monomer composition is essentially free of other polymerization inhibitors, where the content in the monomer composition of p-methoxyphenol in ppm, based on acrylic acid, is equal to $\chi$ or less, where $\chi$ is given by the equation:

$$\chi = 200 - 1.5 \cdot DN,$$

in which DN is the degree of neutralization of the acrylic acid in percent.

**2.** The process according to claim 1, wherein the acrylic acid is present entirely or partly as an alkali metal salt.

**3.** The process according to claim 2, wherein the acrylic acid is present entirely or partly as the sodium and/or potassium salt.

**4.** The process according to any of the preceding claims, wherein the degree of neutralization of the acrylic acid is from 20 to 110 mol%, preferably from 20 to 80 mol%.

**5.** The process according to any of the preceding claims, wherein the composition comprises from 16 to 56% by weight, preferably from 24 to 40% by weight, of monomer, calculated as acrylic acid.

**6.** The process according to any of the preceding claims, wherein the residence time of the monomer composition in the mode of transport or the pipeline is at least 1 hour.

**7.** The process according to any of the preceding claims, wherein the monomer composition is transported as a coherent volume of at least 1 m$^3$.

**8.** The process according to any of the preceding claims, wherein the composition comprises dissolved oxygen and/or is in contact with the ambient air or an oxygen-containing gas space.

**9.** The process according to any of the preceding claims, wherein the monomer composition is suitable for preparing water-absorbing resins.

**Revendications**

**1.** Procédé pour le transport d'une composition de monomères, **caractérisé en ce qu'**on amène une composition, qui comprend une solution aqueuse d'acide acrylique partiellement ou complètement neutralisée ou d'acide acrylique surneutralisé, dans un moyen de transport ou on l'envoie dans une conduite tubulaire, la composition de monomères contenant un inhibiteur de polymérisation sous forme de p-méthoxyphénol et la composition de monomères état sensiblement exempte d'autres inhibiteurs de polymérisation, la teneur de la composition de monomères en p-méthoxyphénol en ppm, par rapport à l'acide acrylique, valant χ ou moins, χ étant donné par l'équation :

$$\chi = 200 - 1{,}5 \cdot DN,$$

dans laquelle DN représente le degré de neutralisation de l'acide acrylique en pour cent.

**2.** Procédé selon la revendication 1, **caractérisé en ce que** l'acide acrylique se trouve totalement ou partiellement sous forme de sel de métal alcalin.

**3.** Procédé selon la revendication 2, **caractérisé en ce que** l'acide acrylique se trouve totalement ou partiellement sous forme de sel de sodium et/ou de potassium.

**4.** Procédé selon l'une quelconque des revendications précédentes, **caractérisé en ce que** le degré de neutralisation de l'acide acrylique vaut 20 à 110% en mole, de préférence 20 à 80% en mole.

**5.** Procédé selon l'une quelconque des revendications précédentes, **caractérisé en ce que** la composition contient 16 à 56% en poids, de préférence 24 à 40% en poids, de monomères, calculés sous forme d'acide acrylique.

**6.** Procédé selon l'une quelconque des revendications précédentes, **caractérisé en ce que** la durée de séjour de la composition de monomères dans le moyen de transport ou la conduite tubulaire est d'au moins 1 heure.

**7.** Procédé selon l'une quelconque des revendications précédentes, **caractérisé en ce qu'**on transporte la composition de monomères sous forme de volume cohérent d'au moins 1 m$^3$.

**8.** Procédé selon l'une quelconque des revendications précédentes, la composition contenant de l'oxygène dissous et/ou se trouvant en contact avec l'air environnant ou avec un espace gazeux contenant de l'oxygène.

**9.** Procédé selon l'une quelconque des revendications précédentes, **caractérisé en ce que** la composition de monomères convient pour la préparation de résines absorbant l'eau.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0020369 A
- WO 03095410 A1
- US 5130471 A
- EP 765856 A
- DE 10219089
- DE 2943707 A
- DE 1205502 C
- EP 117146 A
- EP 293224 A
- GB 1450986 A
- WO 9920590 A
- WO 0053555 A
- DE 2136396 A
- DE 4308087 A
- EP 511111 A
- DE 3429391 A
- JP 1124766 A
- DE 2164767 A
- JP 58140039 A
- US 3553261 A
- US 4219389 A
- GB 1427223 A
- US 3962074 A
- DE 2323328
- US 4493719 A
- EP 616998 A
- EP 648520 A
- EP 715870 A
- EP 776875 A
- WO 9825889 A
- WO 0177056 A
- DE 10221202
- EP 0316518 A
- EP 0202127 A
- DE 19737434
- WO 0065084 A
- WO 0065348 A
- WO 0035502 A

**In der Beschreibung aufgeführte Nicht-Patentliteratur**

- Modern Superabsorbent Polymer Technology. Wiley-VCH, 1998
- Acrylic acid and derivatives, 1.3.1. Propenoxidation. Ullmanns Enzyclopedia of Ind. Chem. Wiley-VCH, 1997
- **K. WEISÄRMEL ; H.-J. ARPE.** Industrielle Org. Chem. VCH Verlagsgesellschaft, 1994, 315-17
- Ullmanns Enzyclopedia of Ind. Chem.